(19) Europäisches Patentamt
European Patent Office
Office européen des brevets

(11) Publication number: **0 049 049**
**B1**

(12) **EUROPEAN PATENT SPECIFICATION**

(45) Date of publication of patent specification: **13.11.85**

(21) Application number: **81303929.4**

(22) Date of filing: **27.08.81**

(51) Int. Cl.[4]: **C 07 D 257/06,**
C 07 D 405/12,
C 07 D 409/12,
C 07 D 403/12, A 61 K 31/41
// C07C121/75, C07C101/12

(54) **5-Amino-tetrazole derivatives, processes for their preparation and pharmaceutical compositions containing them.**

(30) Priority: **27.08.80 GB 8027743**

(43) Date of publication of application:
**07.04.82 Bulletin 82/14**

(45) Publication of the grant of the patent:
**13.11.85 Bulletin 85/46**

(84) Designated Contracting States:
**BE CH DE FR IT LI NL**

(56) References cited:
**EP-A-0 016 565**
**EP-A-0 029 306**

**The file contains technical information submitted after the application was filed and not included in this specification**

(73) Proprietor: **GLAXO GROUP LIMITED**
**Clarges House 6-12 Clarges Street**
**London W1Y 8DH (GB)**

(72) Inventor: **Carey, Linda**
**3 The Lawns Close Melbourn**
**Royston Hertfordshire (GB)**
Inventor: **Clitherow, John Watson**
**54 Gilders**
**Sawbridgeworth Hertfordshire (GB)**
Inventor: **Price, Barry John**
**5 Cowpers Way**
**Tewin Wood Hertfordshire (GB)**
Inventor: **Bradshaw, John**
**22 Whitely Close Dane End**
**Ware Hertfordshire (GB)**
Inventor: **Martin-Smith, Michael**
**Leycroft The Street Haultwick**
**Dane End Ware Hertfordshire (GB)**

(74) Representative: **Marchant, James Ian et al**
**Elkington and Fife High Holborn House 52/54**
**High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 049 049 B1

**Description**

This invention relates to novel heterocyclic derivatives having action on histamine receptors, to processes for the preparation thereof, to pharmaceutical compositions containing them and to their use in therapeutics.

Certain novel heterocyclic derivatives have now been found which have potent activity as $H_2$-antagonists. These compounds, which are more particularly described below, for example show inhibition of the secretion of gastric acid when this is stimulated via histamine receptors (Ash and Schild, Brit. J. Pharmacol. Chemother, 1966, *27*, 427). Their ability to do so can be demonstrated in the perfused rat stomach using the method described in German Offenlegungsschrift No. 2,734,070, modified by the use of sodium pentobarbitone (50 mg/kg) as anaesthetic instead of urethane, and in conscious dogs equipped with Heidenhain pouches using the method described by Black *et al* Nature 1972 *236*, 385. Furthermore the compounds antagonise the effect of histamine on the contraction frequency of isolated guinea pig right atrium but do not modify histamine induced contractions of isolated gastro-intestinal smooth muscle which are mediated via $H_1$-receptors. Certain compounds according to the invention have the advantage of an extended duration of action.

Compounds with histamine $H_2$-blocking activity may be used in the treatment of conditions where there is an advantage in lowering gastric acidicity, particularly in gastric and peptic ulceration, as a prophylactic measure in surgical procedures, and in the treatment of allergic and inflammatory conditions where histamine is a known mediator. Thus they may be used for example, either alone, or in combination with other active ingredients in the treatment of allergic and inflammatory conditions of the skin.

The present invention provides compounds of the general formula (I)

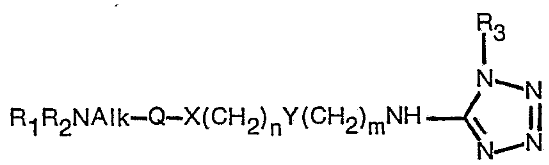

(I)

and physiologically acceptable salts and hydrates thereof in which

$R_1$ represents $C_{1-14}$ alkyl, $C_{3-8}$ cycloalkyl, phenyl $C_{1-6}$ alkyl (optionally substituted by a $C_{1-3}$ alkyl or $C_{1-3}$ alkoxy group or a halogen atom), trifluoro $C_{1-6}$ alkyl, $C_{3-6}$ alkenyl, $C_{3-6}$ alkynyl or $C_{1-6}$ alkyl substituted by hydroxy, $C_{1-6}$ alkoxy, amino, $C_{1-6}$ alkylamino, di $C_{1-6}$ alkylamino or $C_{3-8}$ cycloalkyl; or

$R_1$ represents heteroaralkyl in which the heteroaryl portion is a 5 or 6 membered monocyclic heterocyclic ring containing from 1 to 3 heteroatoms selected from oxygen nitrogen and sulphur, which is unsubstituted or substituted by $C_{1-3}$ alkyl, $C_{1-3}$ alkoxy, hydroxy $C_{1-6}$ alkyl, amino $C_{1-6}$ alkyl, $C_{1-6}$ alkylamino $C_{1-6}$ alkyl, di $C_{1-6}$ alkylamino $C_{1-6}$ alkyl or halogen, linked through a carbon atom to the alkyl portion which is a straight or branched $C_{1-4}$ alkyl chain;

$R_2$ represents hydrogen or a $C_{1-4}$ alkyl group; or

$R_1$ and $R_2$, together with the nitrogen atom to which they are attached, form a 5—10 membered ring which may be saturated or may contain at least one double bond, may be unsubstituted or may be substituted by one or more $C_{1-3}$ alkyl groups, e.g. methyl, or a hydroxy group and/or may contain another heteroatom selected from oxygen or sulphur;

Alk represents a straight or branched $C_{1-6}$ alkylene chain;

Q represents a furan or thiophen ring in which incorporation into the rest of the molecule is through bonds at the 2- and 5-positions, the furan or thiophen ring optionally bearing a further substituent $R_4$ adjacent to the group $R_1R_2NAlk$; or Q represents a thiophen ring in which incorporation into the rest of the molecule is through bonds at the 2- and 4-positions, the thiophen ring optionally bearing a further substituent $R_4$ adjacent to the group $R_1R_2NAlk$ with the proviso that when the group $R_1R_2NAlk$ is in the 4-position then the group $R_4$ is in the 5-position; or Q represents a benzene ring in which incorporation into the rest of the molecule is through bonds at the 1- and 3- or 1- and 4-positions;

$R_4$ represents halogen or $C_{1-4}$ alkyl which may be substituted by hydroxy or $C_{1-4}$ alkoxy;

$R_3$ represents hydrogen, $C_{1-6}$ alkyl, $C_{3-6}$ alkenyl, phenyl $C_{1-6}$ alkyl (optionally substituted by a $C_{1-3}$ alkyl or $C_{1-3}$ alkoxy group or a halogen atom), $C_{2-6}$ alkyl substituted by hydroxy, $C_{1-6}$ alkoxy or $C_{1-4}$ alkanoyloxy;

X and Y, which may be the same or different, each represent oxygen, sulphur, methylene or a bond;

n represents zero, 1, 2 or 3 and m represents an integer from 2 to 5 with the provisos that (a) the total number of atoms in the chain $X(CH_2)_nY(CH_2)_m$ (excluding hydrogen atoms is an integer from 3 to 8 and (b) when X and Y represent oxygen or sulphur then n is 2 or 3.

The term "alkyl" as a group or part of a group refers to a straight or branched chain group and preferably contains 1 to 4 carbon atoms e.g. methyl or ethyl. The heteroaryl ring as part of a heteroaralkyl group may be, for example thienyl, pyrrolyl, pyridyl, furyl or thiazolyl. Thus, for example, the heteroaryl portion of the heteroaralkyl group may be thienyl or furyl substituted by $C_{1-3}$ alkyl, amino $C_{1-6}$ alkyl, $C_{1-6}$ alkylamino $C_{1-6}$ alkyl, di $C_{1-6}$ alkylamino $C_{1-6}$ alkyl or hydroxy $C_{1-6}$ alkyl, pyrrolyl substituted by $C_{1-3}$ alkyl,

pyridyl substituted by $C_{1-3}$ alkyl, $C_{1-3}$ alkoxy, halogen or hydroxy $C_{1-6}$ alkyl or thiazolyl substituted by $C_{1-3}$ alkyl or hydroxy $C_{1-6}$ alkyl.

According to one aspect the invention provides compounds of formula (I) in which Q represents a furan or thiophen ring in which incorporation into the rest of the molecule is through bonds at the 2- and 5-positions, the furan or thiophen ring optionally bearing a further substitutent $R_4$ adjacent to the group $R_1R_2N$-Alk; or Q represents a benzene ring in which incorporation into the rest of the molecule is through bonds at the 1- and 3- or 1- and 4- positions; and

$R_3$ represents hydrogen, $C_{1-6}$ alkyl, $C_{3-6}$ alkenyl, phenyl $C_{1-6}$ alkyl (optionally substituted by a $C_{1-3}$ alkyl or $C_{1-3}$ alkoxy group or a halogen atom) or $C_{2-6}$ alkyl substituted by hydroxy or $C_{1-6}$ alkoxy.

In the compounds according to the invention the chain $X(CH_2)_nY(CH_2)_m$ preferably contains from 4 to 6 atoms (excluding hydrogen atoms). When Q is an optionally substituted furan or thiophene ring the group $X(CH_2)_nY(CH_2)_m$ is preferably $—CH_2O(CH_2)_3—$ or $—(CH_2)_4—$, or more preferably $—CH_2S(CH_2)_2—$. When Q is benzene the group $X(CH_2)_nY(CH_2)_m$ preferably represents $—O(CH_2)_{3-5}$ or $—O(CH_2)_2O(CH_2)_2$. Most preferably Q is a benzene ring in which incorporation into the rest of the molecule is through bonds at the 1- and 3-positions, and the chain $X(CH_2)_nY(CH_2)_m$ is more particularly $—O(CH_2)_{3-4}$.

Alk preferably represents an alkylene chain containing 1 to 4 carbon atoms, e.g. methylene, ethylene or propylene, more preferably methylene.

$R_3$ is preferably $C_{1-4}$ alkyl (e.g. methyl, ethyl or propyl), or hydroxy $C_{2-4}$ alkyl (e.g. hydroxyethyl). More preferably $R_3$ represents $C_{1-4}$ alkyl (e.g. methyl) or hydroxyethyl.

Preferably $R_1$ represents $C_{1-8}$ alkyl (e.g. methyl, ethyl, propyl, butyl, hexyl or heptyl) or a heteroaryl $C_{1-3}$ alkyl group where the heteroaryl portion is as defined above with reference to formula (I) except that the heteroaryl ring contains only one heteroatom (e.g. furylmethyl); and $R_2$ represents hydrogen or methyl; or $R_1R_2N$ represents a saturated 5—7 membered ring optionally containing a double bond or substituted by a hydroxy group (e.g. pyrrolidino, piperidino, tetrahydropyridino or 4-hydroxypiperidino). More preferably $R_1R_2N$ represents di-$C_{1-2}$-alkylamino (e.g. dimethylamino) or a saturated 5—7 membered ring (e.g. piperidino or pyrrolidino).

A particularly preferred group of compounds are those of formula (II)

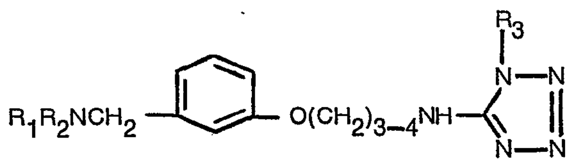

(II)

and physiologically acceptable salts, and hydrates thereof, wherein $R_1R_2N$ represents dimethylamino, piperidino or pyrrolidino; and $R_3$ represents methyl or hydroxyethyl.

Particularly preferred compounds are:

1 - methyl - N - [3 - [3 - (1 - piperidinylmethyl)phenoxy]propyl] - 1*H* - tetrazol - 5 - amine;

1 - methyl - N - [[3 - [3 - (dimethylamino)methyl]phenoxy]propyl] - 1*H* - tetrazol - 5 - amine;

1 - methyl - N - [3 - [3 - (pyrrolidinylmethyl)phenoxy]propyl] - 1*H* - tetrazol - 5 - amine; and

5 - [[3 - [3 - (1 - piperidinylmethyl)phenoxy]propyl]amino] - 1*H* - tetrazol - 1 - (2 - ethanol);

and physiologically acceptable salts and hydrates thereof.

The invention includes the compounds of formula (I) in the form of physiologically acceptable salts with inorganic and organic acids. Particularly useful salts include hydrochlorides, hydrobromides and sulphates, methanesulphonates, acetates, maleates, succinates, tartrates, benzoates, citrates and fumarates. The compounds of formula (I) and their salts may also form hydrates, which hydrates are also to be considered as part of the invention. The compounds of formula (I) can exhibit tautomerism and the formula is intended to cover all tautomers. Where optical isomers may exist the formula is intended to cover all diastereoisomers and optical enantiomers.

The compounds according to the invention, preferably in the form of a salt, may be formulated for administration in any convenient way and the invention includes within its scope pharmaceutical compositions containing at least one compound according to the invention adapted for use in human or veterinary medicine. Such compositions may be formulated in a conventional manner using one or more pharmaceutically acceptable carriers or excipients. Such compositions may also contain if required other active ingredients, e.g. $H_1$-antagonists.

Thus the compounds according to the invention may be formulated for oral, buccal, topical, parenteral or rectal administration. Oral administration is preferred.

For oral administration, the pharmaceutical composition may take the form of for example, tablets, capsules, powders, solutions, syrups or suspensions prepared by conventional means with acceptable excipients. For buccal administration the composition may take the form of tablets or lozenges formulated in conventional manner.

The compounds of the invention may be formulated for parenteral administration by bolus injection or continuous infusion. Formulations for injection may be presented in unit dosage form in ampoules, or in multidose containers, with an added preservative. The compositions may take such forms as suspensions,

solutions or emulsions in oily or aqueous vehicles, and may contain formulatory agents such as suspending, stabilising and/or dispersing agents. Alternatively, the active ingredient may be in powder form for reconstitution with a suitable vehicle, e.g. sterile pyrogen-free water before use.

The compounds of the invention may also be formulated in rectal compositions such as suppositories or retention enemas, e.g. containing conventional suppository bases such as cocoa butter or other glyceride.

For topical application, the compounds of the invention may be formulated as ointments, creams, gels, lotions, powders or sprays in a conventional manner.

For internal administration a convenient daily dosage regime of the compounds according to the invention is 1 to 4 doses to a total of 5 mg to 1 g per day, preferably 5 to 500 mg per day, dependent upon the condition of the patient.

It will be appreciated in the methods for the preparation of the compounds of formula (I) given below that for certain reaction steps it may be necesssary to protect various reactive substituents in the starting materials for a particular reaction and subsequently to remove the protecting group. Such protection and subsequent deprotection may be particularly pertinent when $R_1$ and/or $R_2$ and/or $R_3$ are hydrogen and/or when the substituent $R_3$ is an alkyl group bearing a hydroxy group. Standard protection and deprotection procedures can be employed. For example, an amino group may be protected by formation of a phthalimide group which may subsequently be cleaved by treatment with a hydrazine, e.g. hydrazine hydrate or a primary amine for example methylamine. When $R_3$ is hydrogen, this may be protected by formation of a N-benzyl or N-alkoxyalkyl (e.g. ethoxymethyl) derivative. The N-benzyl group may subsequently be cleaved by hydrogenolysis in the presence of a catalyst e.g. palladium and an alkoxyalkyl derivative may be cleaved by treatment with dilute acid.

In describing the processes which may be used for preparing the compounds of formula (I) or intermediates useful in the preparation thereof any of the groups $R_1$, $R_2$, $R_3$, Alk, Q, X, Y, n and m are as defined in formula (I) unless otherwise stated.

Compounds of formula (I) may be prepared by reducing a compound of formula (III)

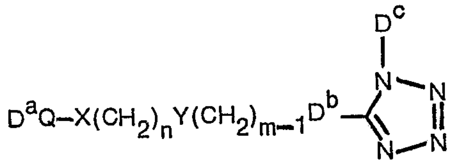

(III)

in which $D^a$ may represent $R_1R_2NAlk$ or a group convertible thereto under reducing conditions such as $R_1R_2NCO$, $R^a_1CONR_2Alk$ (where $R^a_1CO$ represents a group reducible into $R_1$) or CHO; $D^b$ represents —$CH_2$—NH—, —CONH— or —CH=N; and $D^c$ represents $R_3$ or a group convertible thereto under reducing conditions, provided that at least one of $D^a$, $D^b$ and $D^c$ is a reducible group.

In one embodiment of the reduction process, compounds of formula (I) may be prepared by reduction of a compound of formula (III) in which

a) $D^a$ represents $R_1R_2NCO$ or $R^a_1CONR_2Alk$, $D^b$ represents —$CH_2$NH— and $D^c$ represents $R_3$; or

b) $D^a$ represents $R_1R_2NAlk$, $D^b$ represents —CONH— or —CH=N— and $D^c$ represents $R_3$;

with a suitable reducing agent such as a complex metal hydride for example aluminium hydride or lithium aluminium hydride in a solvent such as an ether e.g. tetrahydrofuran or dioxan at a temperature of 20°C to reflux. When the group $D^b$ represents an imino group (—CH=N—) the reduction may also be carried out with a borohydride such as sodium borohydride in a solvent such as an alkanol e.g. ethanol at for example 20°C. Alternatively the reduction may be carried out with hydrogen and a metal catalyst such as palladium or platinum.

In another embodiment of the reduction process compounds of formula (I) in which Alk is —$CH_2$— may be prepared from compounds of formula (III) in which $D^a$ represents —CHO, $D^b$ represents —$CH_2$NH— and $D^c$ represents $R_3$ by reductive alkylation. Thus the compound (III; $D^a$ is CHO) is reacted with ammonia or an amine $R_1R_2NH$ preferably in a solvent such as tetrahydrofuran or an alkanol, e.g. ethanol, followed by reduction. Suitable reducing agents include hydrides such as sodium borohydride or hydrogen and a metal catalyst such as palladium, platinum or Raney nickel, at for example 20°C.

In a further embodiment of the reduction process, a compound of formula (I) in which $R_3$ represents a hydroxyalkyl group may be prepared from a compound of formula (III) in which $D^c$ is a group that may be reduced to a hydroxyalkyl group e.g. an ester, aldehyde or carboxy group, $D^a$ represents $R_1R_2NAlk$ and $D^b$ represents —$CH_2$NH—. Thus for example a compound of formula (III) in which $D^c$ is $(CH_2)_{q-1}CO_2R_5$ where q is an integer from 2 to 6 and $R_5$ is hydrogen, alkyl or aralkyl, may be reduced using for example lithium aluminium hydride under the conditions described above to give a compound of formula (I) in which $R_3$ is the group $(CH_2)_{q-1}CH_2OH$. Compounds of formula (III) in which $D^c$ has the meaning $(CH_2)_{q-1}CHO$ where q is as defined above, may be reduced using sodium borohydride or lithium aluminium hydride or alternatively using hydrogen and a metal catalyst such as palladium or platinum to give a compound of formula (I) in which $R_3$ is the group $(CH_2)_{q-1}CH_2OH$.

In certain instances it is convenient to reduce for example more than one of the groups $D^a$, $D^b$ and $D^c$

simultaneously. Thus for example compounds of formula (III) in which $D^c$ represents $(CH_2)_{q-1}CO_2R_5$, $D^b$ represents CONH and $D^a$ represents $R_1R_2NAlk$ may be reduced using for example lithium aluminium hydride to give compounds of formula (I) in which $R_3$ is the group $(CH_2)_qOH$.

Compounds of formula (III) in which $D^a$ represents $R_1R_2NCO$ or CHO, $D^b$ represents $CH_2NH$ and $D^c$ represents $R_3$ may be prepared by reacting an amine of formula (IV)

$$D^aQX(CH_2)_nY(CH_2)_mNH_2 \qquad (IV)$$

in which $D^a$ represents the group $R_1R_2NCO$ or a protected aldehyde group e.g. an acetal with an aminotetrazole of formula (V)

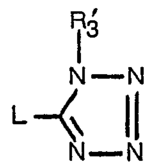

(V)

where L is a leaving group such as halogen e.g. bromine and $R_3'$ is the group $R_3$ or a group convertible thereto.

Similarly compounds of formula (III) in which $D^a$ represents $R_1R_2NAlk$, $D^b$ represents $CH_2NH$ and $D^c$ represents $(CH_2)_{q-1}CHO$ (where the aldehyde grouping is preferably protected as e.g. an acetal) or $(CH_2)_{q-1}CO_2R_5$ (where $R_5$ is hydrogen, alkyl or aralkyl) may be prepared by reacting a diamine of formula (VI)

$$R_1R_2NAlkQX(CH_2)_nY(CH_2)_mNH_2 \qquad (VI)$$

with a tetrazole of the formula (VII)

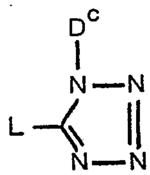

(VII)

where L and $D^c$ are as just defined.

The tetrazoles of formula (VII) are either known compounds or may be prepared by methods analogous to those described in British Patent Specification No. 1364917.

Compounds of formula (III) in which $D^a$ represents $R_1^aCONR_2Alk$ may be prepared by treating the corresponding compounds in which $D^a$ represents $HNR_2Alk$ with an activated derivative of the appropriate acid $R_1^aCO_2H$.

Compounds of formula (III) in which $D^b$ is the group —CONH— may be prepared by reacting the 5-aminotetrazoles (VIII) with an activated derivative of the acid (IX) such as an acid halide, e.g. acid chloride.

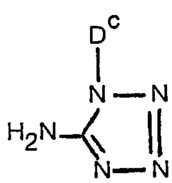

$$R_1R_2NAlkQX(CH_2)_nY(CH_2)_{m-1}CO_2H$$

(VIII) (IX)

Compounds of formula (III) in which $D^b$ represents —CH=N— may be prepared by condensation of the 5-amino-tetrazoles (VIII) with the aldehyde (X)

$$R_1R_2NAlkQX(CH_2)_nY(CH_2)_{m-1}CHO \qquad (X)$$

The 5-aminotetrazoles (VIII) are either known compounds or may be prepared by methods analogous to those described by R. M. Herbst J. Org. Chem. (1951) *16*, 139 and R. A. Henry, J. Amer. Chem. Soc., (1954), *76*, 93.

Compounds of formula (I) may also be prepared by displacement of the group L from a compound of formula (XI)

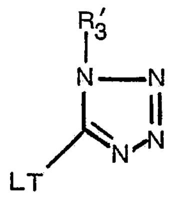

(XI)

in which $R_3'$ is the group $R_3$ or a group convertible thereto, L is a leaving group selected from halogen e.g. chlorine or bromine, or a quaternary ammonium group such as trialkylammonium and T represents a bond, $-AlkQX(CH_2)_nY(CH_2)_mNH-$, $-(CH_2)_mNH-$ or $-(CH_2)_nY(CH_2)_mNH-$, by reaction with a nucleophile of formula (XII)

$$R_1R_2NW \qquad (XII)$$

where W respectively represents $-AlkQX(CH_2)_nY(CH_2)_mNH_2$, hydrogen, $-AlkQX(CH_2)_nYH$ or $-AlkQXH$.

Thus in one embodiment of the above process, compounds of formula (I) may be prepared by reaction of a compound of formula (XI) in which L represents halogen preferably bromine and T represents a bond with a compound of formula (XII) in which W represents $-AlkQX(CH_2)_nY(CH_2)_mNH_2$. The reaction is preferably carried out with heating, for example within the range of 100—200°C, in the absence or presence of a solvent such as ethanol, and preferably in a sealed vessel.

In another embodiment of the above process, compounds of formula (I) may be prepared by reacting a compound of formula (XI) in which L represents a trialkylammonium group such as $^{\ominus}AR^aR^bR^cN^{\oplus}$ in which A is an anion e.g. halide and $R^a$, $R^b$, $R^c$ are each alkyl or aralkyl e.g. $R^aR^bR^cN^{\oplus}$ is trimethylammonium and T represents $-AlkQX(CH_2)_nY(CH_2)_mNH-$ with a compound of formula (XII) in which W represents hydrogen and in which $R_1$ and $R_2$ are other than hydrogen. The reaction is preferably carried out at an elevated temperature, for example 100—150°C.

This embodiment is particularly useful for preparing compounds in which Alk is $CH_2$.

In a further embodiment of this process, compounds of formula (I) in which X is oxygen or sulphur may be prepared from an anion of an alcohol, thiol, phenol or thiophenol, derived from a compound of formula (XII) in which W represents the group $-AlkQX(CH_2)_nYH$ (where Y is oxygen or sulphur) by displacement of the leaving group L from a compound of formula (XI) where L represents halogen preferably chlorine or bromine and T represents $-(CH_2)_mNH-$.

In this reaction the anion derived from the compound of formula (XII) is preferably a phenoxide ion (Q is a benzene ring, X is a bond, n is zero and Y is oxygen). The reaction is carried out by generating the anion, by treating the compound of formula (XII) with a base, e.g. sodium hydride, potassium carbonate or potassium tertiary butoxide in a solvent, e.g. acetone or dimethylformamide at a temperature of 10 to 30°C and then effecting the displacement reaction in the same solvent at temperatures of 25 to 100°C.

Compounds of formula (XI) in which L represents a leaving group such as halogen and T represents a bond are either known compounds or may be prepared by methods analogous to those described in British Patent Specification No. 1,364,917 and G. B. Barlin, J. Chem. Soc., (B), 1967 641.

Compounds of formula (XI) in which L represents a quaternary ammonium group and T represents $-AlkQX(CH_2)_nY(CH_2)_mNH-$ may be prepared by reacting a compound of formula (XIII)

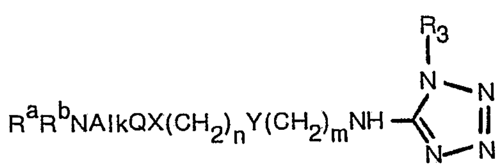

(XIII)

with an alkyl or aralkyl halide e.g. methyliodide or benzyl iodide.

Compounds of formula (XI) in which L represents a leaving group such as halogen and T represents $-(CH_2)_mNH-$ may be prepared by reacting a compound of formula $HO(CH_2)_mNH_2$ with a compound of formula (XI) in which L is a leaving group such as chlorine or bromine and T represents a bond, under the conditions described above for the reaction with a diamine (XII) in which W represents $-AlkQX(CH_2)_nY(CH_2)_mNH_2$, followed by conversion of the hydroxy substituent into a leaving group by standard procedures.

Compounds of formula (I) in which $R_3$ represents a $C_{1-4}$ alkanoyloxy $C_{2-6}$ alkyl group may be prepared by reacting a compound of formula (I) in which $R_3$ represents a hydroxy $C_{2-6}$ alkyl group with a carboxylic acid which corresponds to the $C_{1-4}$ alkanoyloxy group or an activated derivative thereof. Suitable activated derivatives include acid halides e.g. acid chlorides and acid anhydrides. When an acid chloride is used, the reaction is preferably carried out in for example pyridine at room temperature.

Where the product of any of the above processes is a free base and a salt is required, the salt may be formed in a conventional manner. Thus for example, a generally convenient method of forming the salts is

to mix appropriate quantities of the free base and the acid in an appropriate solvent(s) e.g. an alcohol such as ethanol or an ester such as ethyl acetate.

The invention is illustrated but not limited by the following Examples.

In the following Examples and Preparations temperatures are in °C.

Thin layer chromatography (tlc) and column chromatography were carried out on silica using, unless otherwise stated, one of the following solvent systems.

System A. Dichloromethane:ethanol:880 ammonia (150:8:1)

System B. Dichloromethane:ethanol:880 ammonia (100:8:1)

Preparation 1

3-[3-[(Dimethylamino)methyl]phenoxy]propionyl chloride

i) 3-[3-[(Dimethylamino)methyl]phenoxy]propionitrile

A solution of 3-[(dimethylamino)methyl]phenol (201 g), acrylonitrile (477 g) and benzyltrimethylammonium hydroxide (40% methanolic solution, 30 ml) was heated under reflux for 24 h. The mixture was evaporated, diluted with ether (500 ml) and filtered. The filtrate was washed with 2N sodium hydroxide and water, dried and evaporated to give the title compound (148 g) as a pale yellow oil.

Nmr ($CDCl_3$):τ2.72, dd, (1H); 2.9—3.3, m (3H); 5.83, t, (2H); 6.62, t, (2H); 7.24, t, (2H); 7.79, s, (6H).

ii) 3-[3-[(Dimethylamino)methyl]phenoxy]propionic acid

A solution of 3-[3-[(dimethylamino)methyl]phenoxy]propionitrile (147 g) in 2N sulphuric acid (800 ml) was heated under reflux for 96 h. The pH of the cooled solution was adjusted to pH 7 with sodium bicarbonate and ethanol (2000 ml) was added. The mixture was filtered and the filtrate was evaporated and water was removed from the residue by azeotropic distillation with benzene. The resulting oil was triturated with diethylether to give the title compound (110 g) as a white powder, m.p. 86—89°.

iii) 3-[3-[(Dimethylamino)methyl]phenoxy]propionyl chloride

3-[3-[(Dimethylamino)methyl]phenoxy]propionic acid (5.0 g), dichloromethane (120 ml), dimethylformamide (0.5 ml) and thionyl chloride (12 ml) was stirred at room temperature for 3 h. The solvent was evaporated off and water was removed from the residual oil by azeotropic distillation with benzene to give the title compound as a pale yellow foam (5.5 g).

Ir (nujol mull): 1795 $cm^{-1}$

Preparation 2

a) N-[1-Methyl-1*H*-tetrazol-5-yl]-3-[3-(1-piperidinylmethyl)phenoxy]propanamide

A solution of 3-[3-(1-piperidinylmethyl)phenoxy]propionyl chloride (3.75 g) in dimethylformamide (20 ml) was added to 1-methyl-1*H*-tetrazol-5-amine (1.32 g) in dimethylformamide (30 ml). The mixture was stirred at room temperature for 20 h, the solvent was evaporated off to give an oil which was treated with sodium carbonate solution (50 ml) and extracted with ethyl acetate. The extract was washed, dried and evaporated to give the crude product as a gum which was chromatographed using system B to give the title compound (1.6 g) as a pale brown foam.

Nmr ($CDCl_3$):τ1.65, br.s, (1H); 2.83, t, (1H); 3.0—3.3, m, (3H); 5.73, t, (2H); 6.03, s, (3H); 6.53, s, (2H); 7.02, t, (2H); 7.6, m, (4H); 8.5, m, (6H).

b) Similarly prepared from 4-[3-(1-piperidinylmethyl)phenoxy]butyryl chloride (10.7 g) and 1-methyl-1*H*-tetrazol-5-amine (3.6 g), except that the crude product was triturated with cyclohexane and then recrystallised from ethyl acetate, was N - [1 - methyl - 1*H* - tetrazol - 5 - yl] - 4 - [3 - (1 - piperidinylmethyl)phenoxy]butanamide (3 g) as a pale brown powder, m.p. 162—164°.

c) Similarly prepared from 4 - [3 - (1 - piperidinylmethyl)phenoxy]butyryl chloride (1.5 g) and 1 - (1 - methylethyl) - 1*H* - tetrazol - 5 - amine (0.64 g), except that the crude product was recrystallised from methanol, was N - [1 - [1 - (1 - methylethyl)] - 1*H* - tetrazol - 5 - yl] - 4 - [3 - (1 - piperidinylmethyl)phenoxy] butanamide (0.5 g) as white microcrystals, m.p. 132—133°.

d) Similarly prepared from 3 - [3 - (dimethylaminomethyl)phenoxy]propionyl chloride (5.4 g) and 1 - methyl - 1*H* - tetrazol - 5 - amine (2.21 g) was N - [1 - methyl - 1*H* - tetrazol - 5 - yl] - 3 - [3 - [(dimethylamino)methyl]phenoxy]propanamide (2.6 g) as a pale yellow foam.

Nmr ($CDCl_3$):τ0.2, br.s, (1H); 2.8, t, (1H); 3.0—3.3, m., (3H); 5.7, t, (2H); 6.05, s, (3H); 6.55, s, (2H); 7.02, t, (2H); 7.73, s, (6H).

e) Similarly prepared from 3 - [3 - (1 - piperidinylmethyl)phenoxy]propionyl chloride (5.9 g) and 1 - phenylmethyl - 1*H* - tetrazol - 5 - amine (3.7 g) was N - [1 - phenylmethyl - 1*H* - tetrazol - 5 - yl] - 3 - [3 - (1 - piperidinylmethyl)phenoxy]propanamide (2.7 g) as a white powder, m.p. 86—89°C.

Preparation 3

3-[3-[(1-Methyl-1*H*-tetrazol-5-yl)amino]propoxy]benzaldehyde

5 - Bromo - 1 - methyl - 1*H* - tetrazole (10 g), 3 - [3 - (1,3 - dioxolan - 2 - yl)phenoxy]propanamine (15 g) and absolute ethanol (20 ml) were heated in an autoclave at 110° for 8 h. The solvent was evaporated off, the residue treated with excess sodium carbamate solution and extracted with ethyl acetate. The extract (150 ml) was stirred with 2N hydrochloride acid (250 ml) for 1 h and the organic layer was dried and

evaporated to give a brown solid which was recrystallised from ethyl acetate to give the title compound (4.6 g) as a light brown solid, m.p. 91—92°.

Preparation 4

N-[1-ethyl-1*H*-tetrazol-5-yl]-3-[3-(1-piperidinylmethyl)phenoxy]propanamide

The title compound (2 g) was prepared as a light brown oil from 3 - [3 - (1 - piperidinylmethyl)phenoxy]propionyl chloride (5.9 g) and 1 - ethyl - 1*H* - tetrazol - 5 - amine (1.7 g) using the method of preparation 2.

Nmr ($CDCl_3$):2.00, s, (1H); 2.78, dd, (1H); 3.0—3.35, m, (3H); 5.5—5.8, m, (4H); 6.50, s, (2H); 7.03 t, (2H); 7.56, m, (4H); 8.25—8.7, m, (9H).

Example 1

1-Methyl-N-[3-[3-(1-piperidinylmethyl)phenoxy]propyl]-1*H*-tetrazol-5-amine

A solution of N - [1 - methyl - 1*H* - tetrazol - 5 - yl]3 - [3 - (1 - piperidinylmethyl)phenoxy]propanamide (1.4 g) in tetrahydrofuran (100 ml) was added to lithium aluminium hydride (1.4 g) under nitrogen. The mixture was stirred at room temperature for 20 h when water (1.4 ml) was added followed by 15% sodium hydroxide solution (1.4 ml) and more water (4.2 ml). The mixture was filtered and the filtrate was evaporated to give a solid which was recrystallised from methyl acetate-hexane to give the title compound (1.0 g) as a white solid, m.p. 118—119°

| | |
|---|---|
| Analysis Found: | C, 61.5; H, 7.8; N, 25.1 |
| $C_{17}H_{26}N_6O$ requires: | C, 61.8; H, 7.9; N, 25.4% |

b) Similarly prepared from N - [1 - methyl - 1*H* - tetrazol - 5 - yl] - 4 - [3 - (1 - piperidinylmethyl)phenoxy]butanamide (3.1 g), except that dioxan was used as the reaction solvent and the crude product was recrystallised from ether, was 1 - methyl - N - [4 - [3 - (1 - piperidinylmethyl)-phenoxy]butyl] - 1*H* - tetrazol - 5 - amine (1.8 g) as a white powder, m.p. 83—84°.

| | |
|---|---|
| Analysis Found | C, 62.7; H, 8.1; N, 24.1; |
| $C_{18}H_{28}N_6O$ requires: | C, 62.8; H, 8.1; N, 24.4% |

c) Similarly prepared from N - [1 - [1 - (1 - methylethyl)] - 1*H* - tetrazol - 5 - yl] - 4 - [3 - (1 - piperidinylmethyl)phenoxy]butanamide (0.4 g), except that the crude product was chromatographed using system A was 1 - (1 - methylethyl) - N - [4 - [3 - (1 - piperidinylmethyl)phenoxy]butyl] - 1*H* - tetrazol - 5 - amine (54 mg) as a pale brown oil.

Nmr ($CDCl_3$):τ2.76, dd, (1H); 3.0—3.3, m, (3H); 4.82, t, (1H); 5.62, m, (1H); 6.0, t, (2H); 6.45, m, (2H); 6.52, s, (2H); 7.58, m, (4H); 8.10, m, (4H); 8.25—8.70, m+d, (12H).

Tlc system A, $R_f$ 0.5

d) Similarly prepared from N - [1 - methyl - 1*H* - tetrazol - 5 - yl] - 3 - [3 - [(dimethylamino)methyl]phenoxy]propanamide (2.5 g) was 1 - methyl - N - [[3 - [3 - (dimethylamino)-methyl]phenoxy]propyl] - 1*H* - tetrazol - 5 - amine (1.2 g) as white crystals, m.p. 72°.

| | |
|---|---|
| Analysis Found: | C, 57.9; H, 7.6; N, 28.9; |
| $C_{14}H_{22}N_6O$ requires: | C, 57.8; H, 7.7; N, 29.1% |

e) Similarly prepared from N - [1 - phenylmethyl - 1*H* - tetrazol - 5 - yl] - 3 - [3 - (1 - piperidinylmethyl)phenoxy]propanamide (2.5 g) was 1 - phenylmethyl - N - [3 - [3 - (1 - piperidinylmethyl)phenoxy]propyl] - 1*H* - tetrazol - 5 - amine hemihydrate (1.2 g) as white crystals, m.p. 88—90°.

| | |
|---|---|
| Analysis Found: | C, 66.7; H, 7.5; N, 20.5; |
| $C_{23}H_{30}N_6O. \frac{1}{2}H_2O$ requires: | C, 66.5; H, 7.4; N, 20.2% |

Example 2

a) 1-Methyl-N-[3-[3-(pyrrolidinylmethyl)phenoxy]propyl]-1*H*-tetrazol-5-amine hydrate (4:1)

5 - Bromo - 1 - methyl - 1*H* - tetrazole (1.0 g) (Compound A), 3 - [3 - [(1 - pyrrolidinyl)methyl]phenoxy]propanamine (3.58 g) and absolute ethanol (4 ml) were heated at 110° in an autoclave for 48 h. The solvent was evaporated, the residue was basified with excess sodium carbonate and extracted with ethyl acetate. The extract was dried and evaporated to give a yellow solid which was

recrystallised from methyl acetate:petroleum ether (b.p. 60—80°) (1:3) to give the title compound (0.5 g) as white crystals, m.p. 87° (softens).

| | |
|---|---|
| Analysis Found: | C, 60.1; H, 7.3; N, 26.1; |
| $C_{16}H_{24}N_6O.\frac{1}{4}H_2O$ requires: | C, 59.9; H, 7.7; N, 26.2% |

b) Similarly prepared from compound A (1 g) and 2 - [[5 - (dimethylamino)methyl] - 2 - furanylmethyl]thio]ethylamine (3.28 g), except that the reaction was carried out at 120° for 26 h and the crude product was chromatographed using system B to give an oil which was triturated with petroleum ether: methyl acetate (3:1), was 1 - methyl - N - [2 - [[5 - (dimethylamino)methyl - 2 - furanylmethyl]thio]ethyl] - 1*H* - tetrazol - 5 - amine (0.4 g) as a white powder, m.p. 68—69°

Nmr ($CDCl_3$):3.9, s, (2H); 4.87, br.s, (1H); 6.23, s, (3H); 6.3, s, (2H); 6.52, q, (2H); 6.62, s, (2H); 7.17, t, (2H); 7.75, s, (6H).

c) Similarly prepared from compound A (0.8 g) and 3 - [4 - (1 - piperidinyl)methyl]phenoxy]propanamine (0.86 g), except that the reaction was carried out at 125° for 24 h, was 1 - methyl - N - [3 - [4 - (1 - piperidinylmethyl)phenoxy]propyl] - 1*H* - tetrazol - 5 - amine (0.61 g) as a white solid, m.p. 138—139°.

| | |
|---|---|
| Analysis Found: | C, 61.6; H. 7.9; N, 25.2; |
| $C_{17}H_{26}N_6O$ requires: | C, 61.8; H, 7.9; N, 25.4% |

Example 3

1-Methyl-N-[3-[3-(2,3,4,5,6,7-hexahydro-1-azocinylmethyl)phenoxy]propyl]-1*H*-tetrazol-5-amine

i) 3-[3-[(1-methyl-1*H*-tetrazol-5-yl)amino]propoxy]-N,N,N-trimethylbenzenemethanaminium iodide

Methyl iodide (0.33 g) in acetonitrile (1 ml) was added to a solution of 1 - methyl - N - [3 - [3 - (dimethylamino)methyl]phenoxy]propyl - 1*H* - tetrazol - 5 - amine (0.62 g) in acetonitrile (1 ml) and the mixture stirred at room temperature for 2 h. The precipitate was filtered off, washed with acetonitrile and dried to give the title compound (0.38 g) as a white solid.

Nmr ($D_2O$):2.5, t, (1H); 2.85, m, (3H); 5.53, s, (2H); 5.8, t, (2H); 6.24, s, (3H); 6.47, t, (2H); 6.85, s, (9H); 7.85, m, (2H)

ii) 1-Methyl-N-[3-[3-(2,3,4,5,6,7-hexahydro-1-azocinylmethyl)phenoxy]propyl]-1*H*-tetrazol-5-amine

2,3,4,5,6,7-hexahydroazocine (1.41 g) and 3 - [3 - [(1 - methyl - 1*H* - tetrazol - 5 - yl)amino]propoxy] - N,N,N - trimethylbenzenemethanaminium iodide (0.77 g) was heated at 125° for 8 h. The reaction mixture was dissolved in water and extracted with ethyl acetate. The extract was washed, dried and evaporated to give a solid (1.5 g) which was chromatographed using system B to give a white solid (0.25 g) which was recrystallised from methyl acetate:petroleum ether (b.p. 60—80°) to give the title compound (0.10 g) as a white powder, m.p. 108°.

| | |
|---|---|
| Analysis Found: | C, 63.9; H, 8.4; N, 23.5; |
| $C_{19}H_{30}N_6O$ requires: | C, 63.6, H, 8.4; N, 23.4% |

Example 4

5-[[3-[3-(1-piperidinylmethyl)phenoxy]propyl]amino]-1*H*-tetrazol-1-(2-ethanol) hydrate (4:1)

i) 5-[[3-[3-(1-piperidinylmethyl)phenoxy]propyl]amino]-1*H*-tetrazole-1-acetic acid monopotassium salt hemihydrate

5 - Bromo - 1*H* - tetrazole - 1 - acetic acid (1 g), 3 - [3 - (1 - piperidinylmethyl)phenoxy]propanamine (3 g) and ethanol (10 ml) were heated in an autoclave at 100° for 48 h. The mixture was cooled, evaporated, basified with sodium carbonate solution and washed with ethyl acetate. The aqueous phase was saturated with potassium carbonate and extracted with propan-2-ol and the extract was dried ($K_2CO_3$), heated to reflux and filtered hot. The filtrate was cooled, centrifuged and filtered to give a solid which was recrystallised from absolute ethanol to give the title compound (180 mg) as a white powder, m.p. 211—13°

| | |
|---|---|
| Analysis Found: | C, 51.3; H, 6.1; N, 19.6; K, 9.5; |
| $C_{18}H_{25}KN_6O_3.\frac{1}{2}H_2O$ requires: | C, 51.3; H, 6.2; N, 19.9; K, 9.3% |

ii) Ethyl 5-[[3-[3-(1-piperidinylmethyl)phenoxy]propyl]amino]-1*H*-tetrazol-1-acetate hydrate (4:1)

5 - [[3 - [3 - (1 - Piperidinylmethyl)phenoxy]propyl]amino] - 1*H* - tetrazole - 1 - acetic monopotassium salt hemihydrate (1.1 g) and concentrated sulphuric acid (0.2 g) were refluxed in absolute ethanol (60 ml) for 70 h. The mixture was cooled and evaporated, water (50 ml) was added and the solution basified with excess potassium carbonate. The solution was extracted with ethyl acetate and the extract

dried and evaporated to give a gum which was crystallised from methyl acetate—petroleum ether (b.p. 60—80°) to give the title compound (260 mg) as a white powder, m.p. 92—95°.

Analysis Found: C, 59.2; H, 7.5; N, 20.4;

$C_2H_{30}N_6O_3.\frac{1}{4}H_2O$ requires: C, 59.0; H, 7.55; N, 20.6%

iii) 5-[[3-[3-(1-Piperidinylmethyl)phenoxy]propyl]amino]-1*H*-tetrazole-1-(2-ethanol)hydrate (4:1)

Ethyl 5 - [[3 - [3 - (1 - piperidinylmethyl)phenoxy]propyl]amino] - 1*H* - tetrazole - 1 - acetate hydrate (4:1) (200 mg) and lithium aluminium hydride (200 mg) were stirred at room temperature in THF (10 ml) under nitrogen for 1 h. Water (0.2 ml), then 15% sodium hydroxide solution (0.2 ml) and more water (0.6 ml) were then added and the mixture was filtered. The filtrate was evaporated to give a gum which was chromatographed using system B to give a gum which was triturated with ether to give the title compound (70 mg) as a white powder, m.p. 86—87°.

Analysis Found: C, 59.3; H, 7.8; N, 23.3;

$C_{18}N_{28}N_6O_2.\frac{1}{4}H_2O$ requires: C, 59.3; H, 7.9; N, 23.0%

Example 5

The following compounds were prepared according to the method of Example 2:

a) From 5 - bromo - 1 - methyl - 1*H* - tetrazole (compound A) (0.8 g) and 2 - [[5 - (dimethylamino)methyl - 4 - methyl - 2 - furanylmethyl]thio]ethanamine (1.14 g), using the method of Example 2 except that the reaction was carried out at 125° for 20 h and the crude product was chromatographed using system B to give an oil which was triturated with petroleum ether: ether (3:1), N - [2 - [[5 - (dimethylamino)methyl - 4 - methyl - 2 - furanylmethyl]thio]ethyl] - 1 - methyl - 1*H* - tetrazol - 5 - amine (0.28 g) was prepared as a light brown solid, m.p. 172—3°.

Nmr ($CDCl_3$):3.98, s, (1H); 4.63, t, (1H); 6.20, s, (3H); 6.32, s, (2H); 6.50, q, (2H); 6.64, s, (2H); 7.15, t, (2H); 7.75, s, (6H); 8.02, s, (3H).

b) From compound A (0.8 g) and 2 - [[5 - (dimethylamino)methyl - 2 - thienylmethyl]thio]ethanamine (1.14 g), using the method of Example 2 except that the reaction was carried out at 125° for 18 h and the crude product was chromatographed using system B to give an oil which was triturated with ether:petroleum ether (b.p. 60—80°) (1:1), N - [2 - [[5 - (dimethylamino)methyl - 3 - thienylmethyl]thio]ethyl] - 1 - methyl - 1*H* - tetrazol - 5 - amine (0.37 g) was prepared as a white solid, m.p. 54—56°.

Nmr ($CDCl_3$):2.97, s, (1H); 3.12, s, (1H); 5.07, t, (1H); 6.23, s, (3H); 6.32, s, (2H); 6.35, q, (2H); 6.44, s, (2H); 7.22, t, (2H); 7.73, s, (6H).

c) From compound A (0.8 g) and 2 - [2 - [3 - (1 - piperidinylmethyl)phenoxy]ethoxy]ethylamine (1.39 g), using the method of Example 2 except that the reaction was carried out at 125° for 24 h and the crude product was chromatographed using system B to give an oil which was dissolved in ether and treated with excess ethereal hydrogen chloride, 1 - methyl - N - [2 - [2 - [3 - (1 - piperidinylmethyl)-phenoxy]ethoxy]ethyl] - 1*H* - tetrazol - 5 - amine dihydrochloride (0.65 g) was prepared, m.p. 45° (softens).

Analysis Found: C, 49.6; H, 6.9; N, 19.5;

$C_{18}H_{28}N_6O_2$ requires: C, 49.9; H, 7.0; N, 19.4%

Example 6

1-Ethyl-N-[3-[3-(1-piperidinylmethyl)phenoxy]propyl]-1*H*-tetrazol-5-amine hydrate (4:1)

The title compound (0.69 g) was prepared from N - [1 - ethyl - 1*H* - tetrazol - 5 - yl] - 3 - [3 - (1 - piperidinylmethyl)phenoxy]propanamide (1.36 g) using the method of Example 1 as white microcrystals, m.p. 94—95°.

Analysis Found: C, 62.2; H, 8.2; N, 24.0;

$C_{18}H_{28}N_6O.\frac{1}{4}H_2O$ requires: C, 61.9; H. 8.2; N, 24.1%

Example 7

(a) N-[3-[3-[[(2-Furanylmethyl)amino]methyl]phenoxy]propyl]-1-methyl-1*H*-tetrazol-5-amine hydrate (4:1)

3 - [3 - [(1 - Methyl - 1*H* - tetrazol - 5 - yl)amino]propoxy]benzaldehyde (1.5 g) and furfurylamine (7.5 ml) in ethanol (50 ml) were stirred at 21° for 1.5 h. Sodium borohydride (2.0 g) was then added and the reaction stirred for a further 18 h at 21°. Water (100 ml) was added and the mixture evaporated to 25 ml and extracted with ethyl acetate. The extract was dried and evaporated to give an orange oil (2 g) which was

chromatographed using ethyl acetate:methanol (9:1) to give a yellow solid (0.92 g) which was recrystallised from diethyl ether to give the title compound (0.45 g) as a white powder, m.p. 55—56°.

| | |
|---|---|
| Analysis Found: | C, 58.8; H, 6.6; N, 24.5; |
| $C_{17}H_{22}N_6O_2.\frac{1}{4}H_2O$ requires: | C, 58.8; H, 6.5; N, 24.2% |

The following compounds were similarly prepared from 3 - [3 - [(1 - methyl - 1*H* - tetrazol - 5 - yl)amino]propoxy]benzaldehyde (Compound B) and the corresponding amine.

b) Compound B (1.25 g) and hexylamine (6 ml) gave N - [3 - [3 - [(hexylamino)methyl]phenoxy]-propyl] - 1 - methyl - 1*H* - tetrazol - 5 - amine hydrate (4:1) (0.54 g) as an off-white solid, m.p. 96—98°.

| | |
|---|---|
| Analysis Found: | C, 61.2; H, 8.4; N, 23.5; |
| $C_{18}H_{30}N_6O.\frac{1}{4}H_2O$ requires: | C, 61.6; H, 8.75; N, 23.9% |

c) Compound B (0.62 g) and 4-hydroxypiperidine (2.5 g) gave 1 - [[3 - [3 - [(1 - methyl - 1*H* - tetrazol - 5 - yl)amino]propoxy]phenyl]methyl] - 4 - piperidinol hemihydrate (0.37 g) as a white solid, m.p. 72° (softens)

| | |
|---|---|
| Analysis Found: | C, 57.6; H, 7.5; N, 23.3; |
| $C_{17}H_{26}N_6O_2.\frac{1}{2}H_2O$ requires: | C, 57.4; H, 7.3; N, 23.6% |

d) Compound B (0.67 g) and 1,2,5,6-tetrahydropyridine (3 ml) gave 1 - methyl - N - [3 - [3 - [(1,2,5,6 - tetrahydropyridinyl)methyl]phenoxy]propyl] - 1*H* - tetrazol - 5 - amine (0.45 g) as a white solid, m.p. 77° (softens).

| | |
|---|---|
| Analysis Found: | C, 62.2; H, 7.5; N, 25.5; |
| $C_{17}H_{24}N_6O$ requires: | C, 62.2; H. 7.4; N, 25.6% |

Example 8

1-Methyl-N-[3-[3-(1-piperidinylmethyl)phenoxy]propyl]-1*H*-tetrazol-5-amine citrate (1:1) hemihydrate

A solution of citric acid (94 mg) in ethyl acetate (50 ml) was added to a stirred solution of 1 - methyl - N - [3 - [3 - (1 - piperidinylmethyl)phenoxy]propyl] - 1*H* - tetrazol - 5 - amine (143 mg) in ethyl acetate (10 ml) to give a precipitate which was filtered off and washed with ethyl acetate to give the title compound (202 mg) as a white powder, m.p. 35°.

Nmr ($D_2O$) 2.58, t, (1H); 2.8—3.0 m, (3H); 5.8, s, (2H); 5.83, t, (2H); 6.3, s, (3H); 6.45, t, (2H); 6.6, m, (2H); 7.15, m, (2H); 7.2 AB q, (2H); 7.9, m, (2H); 8.0—8.7, m, (6H).

Examples of pharmaceutical compositions

| Tablets | mg/Tablet | mg/Tablet |
|---|---|---|
| Active ingredient | 20.0 | 40.0 |
| Microcrystalline cellulose BPC | 99.5 | 199.0 |
| Magnesium stearate B.P. | 0.5 | 1.0 |
| Compression weight | 120.0 | 240.0 |

The drug is sieved through a 250 μm sieve, blended with the excipients and compressed using 6.5 mm and 8.0 mm diameter punches for the 20 and 40 mg strengths respectively. Tablets of other strengths may be prepared by increasing the compression weight and using punches to suit.

The tablets may be film coated with suitable film forming materials, e.g. methyl cellulose, ethyl cellulose or hydroxypropylmethyl cellulose, using standard techniques. Alternatively the tablets may be sugar coated.

Injection for intravenous administration

| | % w/v |
|---|---|
| Active ingredient | 0.25 |
| Water for Injections BP To | 100.00 |

Sodium chloride may be added to adjust the tonicity of the solution and the pH may be adjusted to that of maximum stability using dilute acid or alkali or suitable buffer salts.

The solution is prepared, clarified and filled under nitrogen into appropriate sized ampoules sealed by fusion of the glass. The injection is sterilised by heating in an autoclave using one of the acceptable cycles. Alternatively the solution may be sterilised by filtration and filled into sterile ampoules under aseptic conditions.

The compounds of Examples 1(a), 1(c), 1(d), 4, 5(b) and 7(b) have $ED_{50}$ values of less than 0.22 mg/kg in the perfused rat stomach test.

The compounds of the invention have in general low toxicity at doses at which they effectively inhibit gastric acid secretion. Thus for example the compounds of the Examples listed above produced no toxic effects when administered intravenously to anaethetised rats at doses of at least 5 times their $ED_{50}$ values.

**Claims**

1. Compounds of the general formula (I)

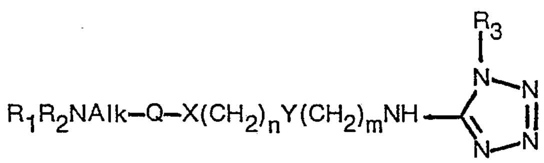

(I)

and physiologically acceptable salts and hydrates thereof in which

$R_1$ represents $C_{1-14}$ alkyl, $C_{3-8}$ cycloalkyl, phenyl $C_{1-6}$ alkyl (optionally substituted by a $C_{1-3}$ alkyl or $C_{1-3}$ alkoxy group or a halogen atom), trifluoro $C_{1-6}$ alkyl, $C_{3-6}$ alkenyl, $C_{3-6}$ alkynyl, or $C_{1-6}$ alkyl substituted by hydroxy, $C_{1-6}$ alkoxy, amino, $C_{1-6}$ alkylamino, di $C_{1-6}$ alkylamino or $C_{3-8}$ cycloalkyl; or

$R_1$ represents heteroaralkyl in which the heteroaryl portion is a 5 or 6 membered monocyclic heterocyclic ring containing from 1 to 3 heteroatoms selected from oxygen, nitrogen and sulphur, which is unsubstituted or substituted by $C_{1-3}$ alkyl, $C_{1-3}$ alkoxy, hydroxy $C_{1-6}$ alkyl, amino $C_{1-6}$ alkyl, $C_{1-6}$ alkylamino $C_{1-6}$ alkyl, di $C_{1-6}$ alkylamino $C_{1-6}$ alkyl or halogen, linked through a carbon atom to the alkyl portion which is a straight or branched $C_{1-4}$ alkyl chain;

$R_2$ represents hydrogen or a $C_{1-4}$ alkyl group; or

$R_1$ and $R_2$ together with the nitrogen atom to which they are attached, form a 5—10 membered ring which may be saturated or may contain at least one double bond, may be unsubstituted or may be substituted by one or more $C_{1-3}$ alkyl groups or a hydroxy group and/or may contain another heteroatom selected from oxygen and sulphur;

Alk represents a straight or branched $C_{1-6}$ alkylene chain;

Q represents a furan or thiophen ring in which incorporation into the rest of the molecule is through bonds at the 2- and 5- positions, the furan or thiophen ring optionally bearing a further substituent $R_4$ adjacent to the group $R_1R_2NAlk$—; or Q represents a thiophen ring in which incorporation into the rest of the molecule is through bonds at the 2- and 4- positions, the thiophen ring optionally bearing a further substituent $R_4$ adjacent to the group $R_1R_2NAlk$ with the proviso that when the group $R_1R_2NAlk$ is in the 4-position then the group $R_4$ is in the 5- position; or Q represents a benzene ring in which incorporation into the rest of the molecule is through bonds at the 1- and 3- or 1- and 4- positions;

$R_4$ represents halogen or $C_{1-4}$ alkyl which may be substituted by hydroxy or $C_{1-4}$ alkoxy;

$R_3$ represents hydrogen, $C_{1-6}$ alkyl, $C_{3-6}$ alkenyl, phenyl $C_{1-6}$ alkyl (optionally substituted by a $C_{1-3}$ alkyl or $C_{1-3}$ alkoxy group or a halogen atom), $C_{2-6}$ alkyl substituted by hydroxy, $C_{1-6}$ alkoxy or $C_{1-4}$ alkanoyloxy;

X and Y, which may be the same or different, each represent oxygen, sulphur, methylene or a bond;

n represents zero, 1, 2 or 3 and m represents an integer from 2 to 5 with the provisos that (a) the total number of atoms in the chain $X(CH_2)_nY(CH_2)_m$ (excluding hydrogen atoms) is an integer from 3 to 8 and (b) when X and Y represent oxygen or sulphur then n is 2 or 3.

2. Compounds as claimed in claim 1 in which the chain $X(CH_2)_nY(CH_2)_m$ contains from 4 to 6 atoms (excluding hydrogen atoms).

3. Compounds as claimed in claim 2 in which the group $X(CH_2)_nY(CH_2)_m$ represents $—O(CH_2)_{3-4}$ and in which Q is a benzene ring in which incorporation into the rest of the molecule is through bonds at the 1- and 3- positions.

4. Compounds as claimed in any of claims 1 to 3 in which Alk is methylene.

5. Compounds as claimed in any of claims 1 to 4 in which $R_3$ is $C_{1-4}$ alkyl or hydroxy $C_{2-4}$ alkyl.

6. Compounds as claimed in any of claims 1 to 5 in which $R_1$ represents $C_{1-8}$ alkyl, or a heteroaryl $C_{1-3}$ alkyl group where the heteroaryl portion is as defined in claim 1 except that the heteroaryl ring contains only one heteroatom; and $R_2$ represents hydrogen or methyl; or $R_1R_2N$ represents a saturated 5 to 7 membered ring optionally containing a double bond or substituted by a hydroxy group.

7. Compounds as claimed in claim 6 in which $R_1R_2N$ is di $C_{1-2}$ alkylamino or a saturated 5 to 7 membered ring.

8. Compounds as claimed in claim 1 in which

Q represents a furan or thiophen ring in which incorporation into the rest of the molecule is through bonds at the 2- and 5- positions, the furan or thiophen ring optionally bearing a further substituent $R_4$ adjacent to the group $R_1R_2N$—Alk—; or Q represents a benzene ring in which incorporation into the rest of the molecule is through bonds at the 1- and 3- or 1- and 4- positions; and

$R_3$ represents hydrogen, $C_{1-6}$ alkyl, $C_{3-6}$ alkenyl, phenyl $C_{1-6}$ alkyl (optionally substituted by a $C_{1-3}$ alkyl or $C_{1-3}$ alkoxy group or a halogen atom) or $C_{2-6}$ alkyl substituted by hydroxy or $C_{1-6}$ alkoxy.

9. Compounds as claimed in claim 1, corresponding to the formula (II)

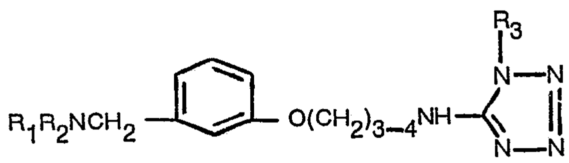

(II)

and physiologically acceptable salts and hydrates thereof, wherein $R_1R_2N$ represents dimethylamino, piperidino or pyrrolidino; and $R_3$ represents methyl or hydroxyethyl.

10. 1 - Methyl - N - [3 - [3 - (1 - piperidinylmethyl)phenoxy]propyl] - 1*H* - tetrazol - 5 - amine and physiologically acceptable salts and hydrates thereof.

11. 1 - Methyl - N - [[3 - [3 - (dimethylamino)methyl]phenoxy]propyl] - 1*H* - tetrazol - 5 - amine and physiologically acceptable salts and hydrates thereof.

12. 1 - Methyl - N - [3 - [3 - (pyrrolidinylmethyl)phenoxy]propyl] - 1*H* - tetrazol - 5 - amine and physiologically acceptable salts and hydrates thereof.

13. 5 - [[3 - [3 - (1 - piperidinylmethyl)phenoxy]propyl]amino] - 1*H* - tetrazol - 1 - (2 - ethanol) and physiologically acceptable salts and hydrates thereof.

14. A process for the preparation of compounds as claimed in claim 1 which comprises

a) reducing a compound of formula (III)

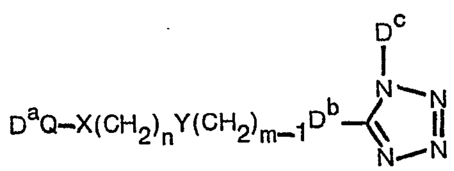

(III)

in which

$D^a$ may represent $R_1R_2N$Alk— or a group convertible thereto under reducing conditions;

$D^b$ represents —$CH_2NH$—, —CONH— or —CH=N; and

$D^c$ represents $R_3$ or a group convertible thereto under reducing conditions, provided that at least one of $D^a$, $D^b$ and $D^c$ is a reducible group; or

b) reacting a compound of formula (XI)

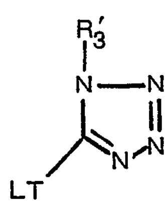

(XI)

in which $R_3'$ is the group $R_3$ or a group convertible thereto, L is a leaving group selected from halogen and quaternary ammonium groups and T represents a bond, —AlkQX$(CH_2)_n$Y$(CH_2)_m$NH—, —$(CH_2)_m$NH— or —$(CH_2)_n$Y$(CH_2)_m$NH—, with a nucleophile of formula (XII)

$$R_1R_2NW \qquad (XII)$$

where W respectively represents —AlkQX$(CH_2)_n$Y$(CH_2)_m$$NH_2$, hydrogen, —AlkQX$(CH_2)_n$YH or —AlkQH; or

c) for the production of compounds of formula (I) in which $R_3$ represents a $C_{1-4}$ alkanoyloxy $C_{2-6}$ alkyl group, reacting a compound of formula (I) in which $R_3$ represents a hydroxy $C_{2-6}$ alkyl group with the carboxylic acid corresponding to the $C_{1-4}$ alkanoyloxy group or an activated derivative thereof; and where the compound of formula (I) is in the form of a free base, optionally converting the free base into a salt.

15. A pharmaceutical composition comprising a compound as claimed in any of claims 1 to 13 and at least one inert pharmaceutically acceptable carrier or diluent, optionally together with at least one other active ingredient.

**Patentansprüche**

1. Verbindungen der allgemeinen Formel (I)

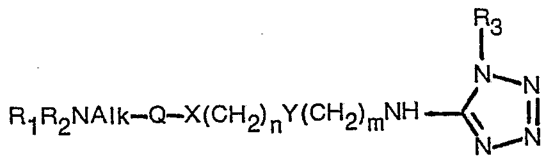

(I)

und ihre physiologisch annehmbaren Salze und Hydrate, worin $R_1$ $C_{1-14}$ Alkyl, $C_{3-8}$-Cycloalkyl, Phenyl-$C_{1-6}$-alkyl (gegebenenfalls durch eine $C_{1-3}$-Alkyl- oder $C_{1-3}$-Alkoxygruppe oder ein Halogenatom substituiert), Trifluor-$C_{1-6}$-alkyl, $C_{3-6}$-Alkenyl, $C_{3-6}$-Alkinyl oder $C_{1-6}$-Alkyl, substituiert durch Hydroxy, $C_{1-6}$-Alkoxy, Amino, $C_{1-6}$-Alkylamino, Di-$C_{1-6}$-alkylamino oder $C_{3-8}$-Cyloalkyl, bedeutet; oder

worin $R_1$ eine Heteroaralkylgruppe bedeutet, in der der Heteroarylteil ein 5- oder 6-gliedriger monocyclischer, heterocylischer Ring, der 1 bis 3 Heteroatome, ausgewählt aus Sauerstoff, Stickstoff und Schwefel, enthält, ist, der unsubstituiert oder durch $C_{1-3}$-Alkyl, $C_{1-3}$-Alkoxy, Hydroxy-$C_{1-6}$-alkyl, Amino-$C_{1-6}$-alkyl, $C_{1-6}$-Alkylamino-$C_{1-6}$-alkyl, Di-$C_{1-6}$-alkylamino-$C_{1-6}$-alkyl oder Halogen substituiert sein kann und über ein Kohlenstoffatom an den Alkylteil, welcher eine geradkettige oder verzweigtkettige $C_{1-4}$-Alkylkette ist, gebunden ist;

$R_2$ Wasserstoff oder eine $C_{1-4}$-Alkylgruppe bedeutet; oder

$R_1$ und $R_2$ zusammen mit dem Stickstoffatom, an das sie gebunden sind, einen 5- bis 10-gliedrigen Ring bilden, der gesättigt sein kann oder der mindestens eine Doppelbindung enthält, der unsubstituiert oder durch eine oder mehrere $C_{1-3}$-Alkylgruppen oder eine Hydroxygruppe substituiert sein kann und/oder der ein weiteres Heteroatom, ausgewählt aus Sauerstoff und Schwefel, enthalten kann;

Alk eine geradkettige oder verzweigte $C_{1-6}$-Alkylenkette bedeutet;

Q einen Furan- oder Thiophenring bedeutet, der an den Rest des Moleküls über die 2- und 5-Stellungen gebunden ist, wobei der Furan- oder Thiophenring gegebenenfalls einen weiteren Substituenten $R_4$ enthält, der zu der Gruppe $R_1R_2$NAlk— benachbart ist; oder Q einen Thiophenring bedeutet, der an den Rest des Moleküls über die 2- und 4-Stellungen gebunden ist, wobei der Thiophenring gegebenenfalls einen weiteren Substituenten $R_4$ aufweist, der benachbart zu der Gruppe $R_1R_2$NAlk ist, mit der Maßgabe, daß, wenn die Gruppe $R_1R_2$NAlk in 4-Stellung steht, die Gruppe $R_4$ in 5-Stellung steht;

oder Q einen Benzolring bedeutet, der an den Rest des Moleküls durch Bindungen in den 1- und 3- oder 1- und 4-Stellungen gebunden ist;

$R_4$ Halogen oder $C_{1-4}$-Alkyl bedeutet, welches durch Hydroxy oder $C_{1-4}$-Alkoxy substituiert sein kann;

$R_3$ Wasserstoff, $C_{1-6}$-Alkyl, $C_{3-6}$-Alkenyl, Phenyl-$C_{1-6}$-alkyl (gegebenenfalls durch eine $C_{1-3}$-Alkyl- oder $C_{1-3}$-Alkoxygruppe oder ein Halogenatom substituiert), $C_{2-6}$-Alkyl, substituiert durch Hydroxy, $C_{1-6}$-Alkoxy oder $C_{1-4}$-Alkanoylalkoxy, bedeutet;

X und Y, die gleich oder unterschiedlich sein können, je Sauerstoff, Schwefel, Methylen oder eine Bindung bedeuten;

n 0, 1, 2 oder 3 bedeutet und m eine ganze Zahl von 2 bis 5 bedeutet, mit den Maßgaben, daß (a) die Gesamtzahl der Atome in der Kette $X(CH_2)_nY(CH_2)_m$ (ausgenommen Wasserstoffatome) eine ganze Zahl von 3 bis 8 beträgt und (b), wenn X und Y Sauerstoff oder Schwefel bedeuten, n 2 oder 3 bedeutet.

2. Verbindungen nach Anspruch 1, dadurch gekennzeichnet, daß die Kette $X(CH_2)_nY(CH_2)_m$ 4 bis 6 Atome (ausgenommen Wasserstoffatome) enthält.

3. Verbindungen nach Anspruch 2, dadurch gekennzeichnet, daß die Gruppe $X(CH_2)_nY(CH_2)_m$ $—O(CH_2)_{3-4}$ bedeutet und daß Q ein Benzolring ist, der an den Rest des Moleküls über Bindungen in den 1- und 3-Stellungen gebunden ist.

4. Verbindungen nach einem der Ansprüche 1 bis 3, dadurch gekennzeichnet, daß Alk Methylen bedeutet.

5. Verbindungen nach einem der Ansprüche 1 bis 4, dadurch gekennzeichnet, daß $R_3$ $C_{1-4}$-Alkyl oder Hydroxy-$C_{2-4}$-alkyl bedeutet.

6. Verbindungen nach einem der Ansprüche 1 bis 5, dadurch gekennzeichnet, daß $R_1$ $C_{1-8}$-Alkyl oder eine Heteroaryl-$C_{1-3}$-alkylgruppe bedeutet, worin der Heteroarylteil die in Anspruch 1 angegebene Definition besitzt, ausgenommen daß der Heteroarylteil nur ein Heteroatom enthält; und $R_2$ Wasserstoff oder Methyl bedeutet; oder $R_1R_2N$ einen gesättigten 5- bis 7-gliedrigen Ring bedeutet, der gegebenenfalls eine Doppelbindung enthält oder durch eine Hydroxygruppe substituiert ist.

7. Verbindungen nach Anspruch 6, dadurch gekennzeichnet, daß $R_1R_2N$ Di-$C_{1-2}$-alkylamino bedeutet oder ein gesättigter 5- bis 7-gliedriger Ring ist.

8. Verbindungen nach Anspruch 1, dadurch gekennzeichnet, daß

Q einen Furan- oder Thiophenring bedeutet, der an den Rest des Moleküls über Bindungen in den 2- und 5-Stellungen gebunden ist, wobei der Furan- oder Thiophenring gegebenenfalls einen weiteren Substituenten $R_4$ aufweist, der zu der Gruppe $R_1R_2N$—Alk— benachbart ist; oder Q einen Benzolring bedeutet, der an den Rest des Moleküls durch Bindungen in den 1- und 3- oder 1- und 4-Stellungen gebunden ist; und

$R_3$ Wasserstoff, $C_{1-6}$-Alkyl, $C_{3-6}$-Alkenyl, Phenyl-$C_{1-6}$-alkyl (gegebenenfalls durch eine $C_{1-3}$-Alkyl- oder $C_{1-3}$-Alkoxygruppe oder ein Halogenatom substituiert) oder $C_{2-6}$-Alkyl, substituiert durch Hydroxy oder $C_{1-6}$-Alkoxy, bedeutet.

9. Verbindungen nach Anspruch 1, entsprechend der Formel (II)

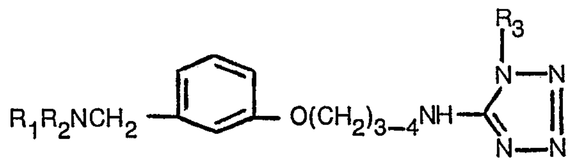

(II)

und ihre physiologisch annehmbaren Salze und Hydrate, worin $R_1R_2N$ Dimethylamino, Piperidino oder Pyrrolidino bedeutet; und $R_3$ Methyl oder Hydroxyethyl bedeutet.

10. 1 - Methyl - N - [3 - [3 - (1 - piperidinylmethyl)phenoxy]propyl] - 1*H* - tetrazol - 5 - amin und seine physiologisch annehmbaren Salze und Hydrate.

11. 1 - Methyl - N - [[3 - [3 - (dimethylamino)methyl]phenoxy]propyl] - 1*H* - tetrazol - 5 - amin und seine physiologisch annehmbaren Salze und Hydrate.

12. 1 - Methyl - N - [3 - [3 - (pyrrolidinylmethyl)phenoxy]propyl] - 1*H* - tetrazol - 5 - amin und seine physiologisch annehmbaren Salze und Hydrate.

13. 5 - [[3 - [3 - (1 - Piperidinylmethyl)phenoxy]propyl]amino] - 1*H* - tetrazol - 1 - (2 - ethanol) und seine physiologisch annehmbaren Salze und Hydrate.

14. Verfahren zur Herstellung von Verbindung nach Anspruch 1, dadurch gekennzeichnet, daß man

a) eine Verbindung der Formel (III)

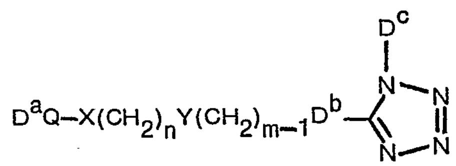

(III)

worin

$D^a$ $R_1R_2NAlk$— oder eine darin unter Reduktionsbedingungen überführbare Gruppe bedeutet;

$D^b$ —$CH_2NH$—, —CONH— oder —CH=N bedeutet; und

$D^c$ $R_3$ oder eine darin unter Reduktionsbedingungen überführbare Gruppe bedeutet, mit der Maßgabe, daß mindestens einer der Substituenten $D^a$, $D^b$ und $D^c$ eine reduzierbare Gruppe bedeutet; reduziert oder

b) eine Verbindung der Formel (XI)

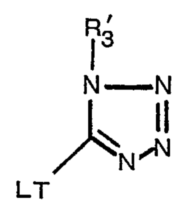

(XI)

worin $R_3'$ die Gruppe $R_3$ oder eine darin überführbare Gruppe bedeutet, L eine abspaltbare Gruppe, ausgewählt aus Halogen und quaternären Ammoniumgruppen, und T eine Bindung, —$AlkQX(Ch_2)_nY(CH_2)_mNH$—, —$(CH_2)_mNH$ oder —$(CH_2)_nY(CH_2)_mNH$— bedeuten, mit einem Nucleophil der Formel (XII)

$$R_1R_2NW \qquad (XII)$$

worin W —$AlkQX(CH_2)_nY(CH_2)_mNH_2$, Wasserstoff, —$AlkQX(CH_2)_nYH$ oder —AlkQH bedeutet; umsetzt oder

c) zur Herstellung von Verbindungen der Formel (I), worin $R_3$ eine $C_{1-4}$-Alkanoyloxy-$C_{2-6}$-alkylgruppe bedeutet, eine Verbindung der Formel (I), worin $R_3$ eine Hydroxy-$C_{2-6}$-alkylgruppe bedeutet, mit einer Carbonsäure entsprechend der $C_{1-4}$-Alkanoyloxygruppe oder einem aktivierten Derivat davon umsetzt; und

wenn die Verbindung der Formel (I) in Form einer freien Base vorliegt, gegebenenfalls die freie Base in ein Salz überführt.

15. Pharmazeutische Zubereitung, dadurch gekennzeichnet, daß sie eine Verbindung nach einem der Ansprüche 1 bis 13 und mindestens einen inerten pharmazeutisch annehmbaren Träger oder Verdünnungsmittel, gegebenenfalls zusammen mit mindestens einem anderen aktiven Bestandteil, enthält.

**Revendications**

1. Composés de formule générale (I)

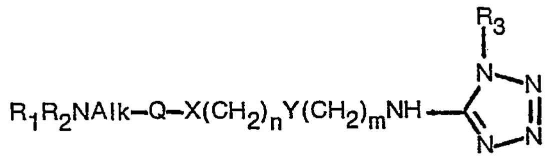

(I)

et leurs sels et hydrates physiologiquement acceptables dans lesquels

$R_1$ représente un groupe alkyle en $C_{1-14}$, cycloalkyle en $C_{3-8}$, phényl(alkyle en $C_{1-6}$) (éventuellement substitué par un groupe alkyle en $C_{1-3}$ ou alcoxy en $C_{1-3}$ ou par un atome d'halogène), trifluoroalkyle en $C_{1-6}$, alcényle en $C_{3-6}$, alcynyle en $C_{3-6}$ ou alkyle en $C_{1-6}$ substitué par un groupe hydroxy, alcoxy en $C_{1-6}$, amino, (alkyl en $C_{1-6}$)amino, di(alkyl en $C_{1-6}$) amino ou cycloalkyle en $C_{3-8}$; ou

$R_1$ représente un groupe hétéroarylalkyle dans lequel la partie hétéroaryle est un hétérocycle à 5 ou 6 chaînons monocyclique contenant de 1 à 3 hétéroatomes choisis parmi l'oxygène, l'azote et le soufre, qui est non substitué ou substitué par au moins un groupe alkyle en $C_{1-3}$, alcoxy en $C_{1-3}$, hydroxyalkyle en $C_{1-6}$, aminoalkyle en $C_{1-6}$, (alkyl en $C_{1-6}$)amino(alkyle en $C_{1-6}$), di(alkyl en $C_{1-6}$)amino(alkyle en $C_{1-6}$) ou halogène, relié à la partie alkyle, qui est une chaîne alkyle en $C_{1-4}$ droite ou ramifiée, par un atome de carbone;

$R_2$ représente l'hydrogène ou un groupe alkyle en $C_{1-4}$; ou

$R_1$ et $R_2$ forment ensemble, avec l'atome d'hydrogène auquel ils sont fixés, un cycle à 5—10 chaînons qui peut être saturé ou peut contenir au moins une double liaison, peut être non substitué ou peut être substitué par un ou plusieurs groupes alkyle en $C_{1-3}$ ou par un groupe hydroxy et/ou peut contenir un autre hétéroatome choisi parmi l'oxygène et le soufre;

Alk représente une chaîne alkylène en $C_{1-6}$ droite ou ramifiée;

Q représente un cycle furanne ou thiophène dans lequel l'incorporation au reste de la molécule se fait par des liaisons en positions 2 et 5, le cycle furanne ou thiophène comportant éventuellement un autre substituant $R_4$ adjacent au groupe $R_1R_2NAlk—$; ou Q représente un cycle thiophène dans lequel l'incorporation au reste de la molécule se fait par des liaisons en positions 2 et 4, le cycle thiphène comportant éventuellement un autre substituant $R_4$ adjacent au groupe $R_1R_2NAlk$, à condition que, lorsque le groupe $R_1R_2NAlk$ est en position 4, le groupe $R_4$ soit en position 5; ou Q représente un noyau benzénique dans lequel l'incorporation au reste de la molécule se fait par des liaisons en positions 1 et 3 ou 1 et 4;

$R_4$ représente un halogène ou un groupe alkyle en $C_{1-4}$ qui peut être substitué par un groupe hydroxy ou alcoxy en $C_{1-4}$;

$R_3$ représente l'hydrogène, un groupe alkyle en $C_{1-6}$, alcényle en $C_{3-6}$, phényl(alkyle en $C_{1-6}$) (éventuellement substitué par un groupe alkyle en $C_{1-3}$ ou alcoxy en $C_{1-3}$ ou par un atome d'halogène), alkyle en $C_{2-6}$ substitué par un groupe hydroxy, alcoxy en $C_{1-6}$ ou alcanoyloxy en $C_{1-4}$;

X et Y qui peuvent être identiques ou différents, représentent chacun l'oxygène, le soufre, le méthylène ou une liaison;

n représente zéro, 1, 2 ou 3 et m représente un nombre entier de 2 à 5, à condition que (a) le nombre total d'atomes de la chaîne $X(CH_2)_nY(CH_2)_m$ (à l'exclusion des atomes d'hydrogène) soit un nombre entier de 3 à 8 et que (b) lorsque X et Y représentent l'oxygène ou le soufre, n soit égal à 2 ou 3.

2. Composés selon la revendication 1, dans lesquels la chaîne $X(CH_2)_nY(CH_2)_m$ contient de 4 à 6 atomes (à l'exclusion des atomes d'hydrogène).

3. Composés selon la revendication 2, dans lesquels le groupe $X(CH_2)_nY(CH_2)_m$ représente $—O(CH_2)_{3-4}$ et dans lesquels Q est un noyau benzénique dans lequel l'incorporation au reste de la molécule se fait par des liaisons en positions 1 et 3.

4. Composés selon l'une quelconque des revendications 1 à 3, dans lesquels Alk est le méthylène.

5. Composés selon l'une quelconque des revendications 1 à 4, dans lesquels $R_3$ est un groupe alkyle en $C_{1-4}$ ou hydroxyalkyle en $C_{2-4}$.

6. Composés selon l'une quelconque des revendications 1 à 5, dans lesquels $R_1$ représente un groupe alkyle en $C_{1-8}$ ou hétéroaryl(alkyle en $C_{1-3}$) dans lequel la partie hétéroaryle est telle que définie dans la revendication 1, sauf que le cycle hétéroaryle ne contient qu'un hétéroatome; et $R_2$ représente l'hydrogène ou le méthyle; ou $R_1R_2N$ représente un cycle saturé comportant de 5 à 7 chaînons et contenant éventuellement une double liaison ou substitué par un groupe hydroxy.

7. Composés selon la revendication 6, dans lesquels $R_1R_2N$ est un groupe di(alkyl en $C_{1-2}$)amino ou un cycle saturé comportant 5 à 7 chaînons.

8. Composés selon la revendication 1, dans lesquels

Q représente un cycle furanne ou thiophène dans lequel l'incorporation au reste de la molécule se fait par des liaisons en positions 2 et 5, le cycle furanne ou thiophène comportant éventuellement un ature substituant $R_4$ adjacent au groupe $R_1R_2NAlk—$; ou Q représente un noyau benzénique dans lequel l'incorporation au reste de la molécule se fait par des liaisons en positions 1 et 3 ou 1 et 4; et

$R_3$ représente l'hydrogène, un groupe alkyle en $C_{1-6}$, alcényle en $C_{3-6}$, phényl(alkyle en $C_{1-6}$) (éventuellement substitué par un groupe alkyle en $C_{1-3}$ ou alcoxy en $C_{1-3}$ ou par un atome d'halogène) ou alkyle en $C_{2-6}$ substitué par un groupe hydroxy ou alcoxy en $C_{1-6}$.

9. Composés selon la revendication 1, correspondant à la formule (II)

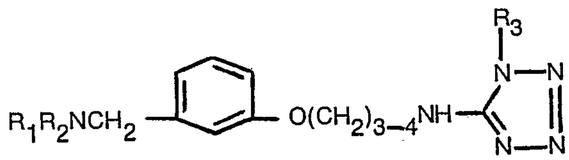

(II)

et leurs sels et hydrates physiologiquement acceptables, dans lesquels $R_1R_2N$ représente le groupe diméthylamino, pipéridino ou pyrrolidino; et $R_4$ représente le groupe méthyle ou hydroxyéthyle.

10. 1 - Méthyl - N - [3 - [3 - (1 - pipéridinylméthyl)phénoxy]propyl] - 1H - tétrazol - 5 - amine et ses sels et hydrates physiologiquement acceptables.

11. 1 - Méthyl - N - [[3 - [3 - diméthylamino)méthyl]phénoxy]propyl] - 1H - tétrazol - 5 - amine et ses sels et hydrates physiologiquement acceptables.

12. 1 - Méthyl - N - [3 - [3 - (1 - pyrrolidinylméthyl)phénoxy]propyl] - 1H - tétrazol - 5 - amine et ses sels et hydrates physiologiquement acceptables.

13. 5 - [[3 - [3 - (1 - Pipéridinylméthyl)phénoxy]propyl]amino] - 1H - tétrazol - 1 - (2 - éthanol) et ses sels et hydrates physiologiquement acceptables.

14. Procédé pour la préparation de composés selon la revendication 1 qui consiste à

a) réduire un composé de formule (III)

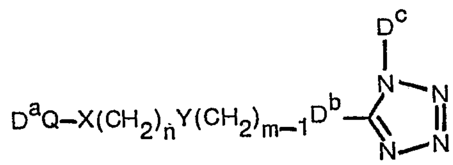

(III)

dans laquelle

$D^a$ peut représenter $R_1R_2NAlk$— ou un groupe pouvant être transformé en celui-ci dans des conditions réductrices;

$D^b$ représente —$CH_2NH$—, —CONH— ou —CH=N; et

$D^c$ représente $R_3$ ou un groupe pouvant être transformé en celui-ci dans des conditions réductrices, à condition que l'un au moins de $D^a$, $D^b$ et $D^c$ soit un groupe réductible; ou

b) faire réagir un composé de formule (XI)

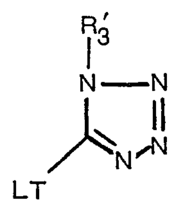

(XI)

dans laquelle $R_3'$ est le groupe $R_3$ ou un groupe pouvant être transformé en celui-ci, L est un groupe partant choisi parmi les groupes halogène et ammonium quaternaire et T représente une liaison, —$AlkQX(CH_2)_nY(CH_2)_mNH$—, —$(CH_2)_mNH$— ou —$(CH_2)_nY(CH_2)_mNH$—, avec un nucléophile de formule (XII)

$R_1R_2NW$ (XII)

dans laquelle W représente respectivement —$AlkQX(CH_2)_nY(CH_2)_mNH_2$, l'hydrogène, —$AlkQX(CH_2)_nYH$ ou —AlkQH; ou

c) pour la production de composés de formule (I) dans laquelle $R_3$ représente un groupe (alcanoyloxy en $C_{1-4}$)alkyle en $C_{2-6}$, faire réagir un composé de formule (I) dans laquelle $R_3$ représente un groupe hydroxyalkyle en $C_{2-6}$ avec l'acide carboxylique correspondant au groupe alcanoyloxy en $C_{1-4}$ ou un de ses dérivés activés;

et lorsque le composé de formule (I) se présente sous forme de base libre, transformer éventuellement la base libre en un sel.

15. Composition pharmaceutique contenant un composé selon l'une quelconque des revendications 1 à 13 et au moins un véhicule ou diluant inerte pharmaceutiquement acceptable, éventuellement avec au moins une autre substance active.